# EUROPEAN PATENT APPLICATION

(11) **EP 3 701 876 A1**
(43) Date of publication of application: **02.09.2020**
(21) Application number: 19159797.0
(22) Date of filing: 27.02.2019
(51) Int. Cl.: A61B 8/08, A61B 7/02, A61B 5/00

(54) **ACOUSTIC SENSING APPARATUS AND METHOD**

(71) Applicant: Koninklijke Philips N.V., 5656 AG Eindhoven (NL)
(72) Inventor: HIWALE, Sujitkumar Sureshrao, 5656 AE Eindhoven (NL); BERA, Deep, 5656 AE Eindhoven (NL); DE WILD, Nico Maris Adriaan, 5656 AE Eindhoven (NL); CHAKRABARTI, Biswaroop, 5656 AE Eindhoven (NL); GIJSBERS, Gerardus Henricus Maria, 5656 AE Eindhoven (NL); RAMACHANDRAN, Ganesan, 5656 AE Eindhoven (NL); JOHNSON, Mark Thomas, 5656 AE Eindhoven (NL)
(74) Representative: de Haan, Poul Erik

(57) **Abstract**

An acoustic sensing apparatus (12) and method is for acoustically surveying the chest area of a subject, and in particular the rib cage. The apparatus includes an arrangement (16) of one or more sound sensors (18) and a controller (24), wherein the arrangement is configured to be placed on the chest of the subject in use, and to detect acoustic signals received from inside the chest. Based on the signal intensities picked up at a plurality of different locations across the chest, the different locations are each classified as being positioned either above a rib of the subject, or above an intercostal space of the subject. In a set of embodiments, the sound sensors are configured to sense sounds generated inside the body (e.g. heart and/or lung sounds, or vocal sounds). In a further set of embodiments, a sound generator may be provided for generating sound signals for transmission into the chest.

## Description

### FIELD OF THE INVENTION

The invention provides an acoustic sensing apparatus and method, in particular for use in surveying positions of ribs of a subject.

### BACKGROUND OF THE INVENTION

A common type of clinical patient examination is cardiovascular examination.

A cardiovascular examination often comprises examination not only of a patient's heart, but also examination of other parts of the body including the hands, face and neck. The cardiovascular examination aims to identify any cardiovascular pathology that may be causing a patient's symptoms, such as chest pain, breathlessness, or heart failure.

Key observations which may be performed during the physical examination of the heart include: measurement of heart rate; measurement of a size of the heart (e.g. measured by percussion and feeling the ictus of the heart) for instance as an indication for enlargement of the left ventricle; and inspection of heart valve function and blood flow, for instance observed via auscultation of the heart at four standard positions, related to the different heart valves, these being the mitral valve, aortic valve, tricuspid valve, and pulmonary valve. Heart sounds and murmurs give indications for valve defects, volume overload, pressure overload and hypertrophy.

One modality which can be used for performing cardiovascular examination is echocardiography.

An echocardiogram is an ultrasound test which can be used for evaluating structures of the heart, as well as the direction of blood flow within the heart. Technicians specially trained in echocardiography perform the scan using an ultrasound probe to produce images and videos, often using a special probe or transducer that is placed in various places on the chest wall, to view the heart from different directions. Cardiologists, or heart specialists, are trained to evaluate the acquired images to assess heart function and provide a report of the results.

Information produced by an echocardiogram may provide indication of one or more of the following:
- Heart size. Weakened or damaged heart valves, high blood pressure, or other diseases can cause the chambers of the heart to enlarge or the walls of the heart to become abnormally thickened.
- Heart pumping strength. An echocardiogram can assist in determining the heart's pumping strength. Specific measurements may include a percentage of blood evacuated from a filled ventricle during each heartbeat (ejection fraction) or a volume of blood pumped by the heart in one minute (cardiac output).
- Damage to the heart muscle. During an echocardiogram, it is possible to determine whether all parts of the heart wall are contributing normally to heart pumping activity. Parts that exhibit weak movement may have been damaged during a heart attack or be receiving too little oxygen. This may indicate coronary artery disease or various other conditions.
- Valve problems. An echocardiogram indicates movement of heart valves as the heart beats. It can be determined from this if the valves open sufficiently wide for adequate blood flow (i.e. no stenosis) and/or close fully for preventing blood leakage (i.e. no valve regurgitation).
- Heart defects. Many heart defects may be detected with an echocardiogram, including problems with heart chambers, abnormal connections between the heart and major blood vessels, and complex heart defects that may be present at birth. Echocardiograms can also be used to monitor a baby's heart development before birth.

In addition to the above, it is also possible (using more advanced analysis techniques) to assess heart wall thickness, wall kinetics and blood flow patterns.

Various different hardware implementations for ultrasound examinations exist.

The most common approach takes the form of an ultrasound probe having an array of ultrasound transducers acoustically coupled to a skin contact area located at its tip. This is slid across the patient's skin, typically using acoustic coupling gel applied between the skin and the probe. The ultrasound probe may be a handheld probe device connected to an ultrasound imaging system or device, for instance mounted in the form of a cart or trolley.

Ultrasound probes utilize ultrasound transducers to generate the acoustic signals. Different types of ultrasound transducer exist. The most common type of transducer are piezoelectric transducers.

One alternative, and advantageous, type are capacitive micromachined ultrasonic transducers (CMUT). CMUT transducers are a relatively recent development. CMUTs utilize changes in capacitance for providing the energy transduction function. CMUTs are constructed on silicon using micromachining techniques. A cavity is formed in a silicon substrate, and a thin membrane suspended over the cavity on which a metallized layer acts as an electrode. The silicon substrate serves as a lower electrode.

As CMUTs are micromachined devices, it is simpler using this technology to construct 2D and 3D arrays of transducers. This means large numbers of CMUTs can be included in a transducer array, providing larger bandwidth compared to other transducer technologies.

Furthermore, achieving high frequency operation is also easier using CMUTs due to their smaller dimensions. The frequency of operation depends upon the transducer cell size (in particular, the size of the cavity covered by the membrane), and also upon the stiffness of the material used for the membrane.

Furthermore, as CMUT transducers are constructed on silicon, integration of additional control or driving electronics is also easier compared to other transducer technologies. This provides the potential for reducing form factor of a device by integrating control components on the same chip as the transducers for instance.

PMUT transducers may also be used in alternative examples.

An alternative hardware approach is use of an electronic stethoscope, which assists in performing cardiac auscultation. Recent developments have been made in this area for more sophisticated processing of auscultatory heart sound signals to enable for example improved analysis and clarification of the resulting sounds, for enabling diagnosis based on the results.

With advancements in communication technology and computerization, remote monitoring has become possible. Due to difficulty in bringing a patient to hospital or due to non-availability of doctors, on many occasions a remote monitoring of physical parameters is preferable. Additionally, hand-free autonomous monitoring in general is also beneficial, for instance in cases where a clinician requires cardiac information in parallel with performing another clinical operation or examination with the patient.

However, where the doctor is not present, or not attentive to the examination - or alternatively for staff with limited specialist experience - accurate placement of the monitoring device can be problematic. If the sensors are not positioned correctly for collection of data, sounds from the important cardiac regions may not be properly obtained.

Means for addressing this problem would therefore be of value.

### SUMMARY OF THE INVENTION

The invention is defined by the claims.

According to examples in accordance with an aspect of the invention, there is provided an acoustic sensing apparatus for sensing rib positions of a subject, the apparatus for placement in use at one or more locations on a chest of the subject, the apparatus comprising:
an arrangement of one or more sound sensors adapted to sense sound signals from the chest of the subject, and
a controller adapted to:
   process sound signals received in use at the arrangement of one or more sound sensors at a plurality of different receiving locations across the chest of the subject, and to classify each of the different receiving locations as either rib-aligned or intercostal space-aligned (gaps between ribs), based on an intensity of sound signals received at each of the locations.

Embodiments of the invention are based on using acoustic signals to perform position sensing. A position of the apparatus relative to the subject's ribs can be detected based on analysis of sensed sound signals which have been received from the chest, via the rib cage area. The ribs and intercostal spaces between the ribs cause respectively different effects on the signal intensity, with the ribs causing attenuation of sounds originating from inside the chest, and augmentation of sounds originating from the chest surface, while the spaces cause little or no attenuation or augmentation of such sounds. This allows a mapping or surveying of the rib area based on intensity of received sound signals.

Using this information, optionally the sound signals may be appropriately processed or analyzed to extract the signal parts originating from the relevant anatomical locations within the chest.

Use of acoustic sensing is a simple mechanism, and enables use of integrated sound sensors which would typically already be present in a remote cardiac monitoring device. Hence duplication of parts can be avoided. The apparatus may in some cases form part of, or may be, a cardiac monitoring device for performing cardiac examination of the subject.

Two main implementation options are possible.

In one main set of embodiments, the apparatus is provided having just a single sound sensor, or a small patch of sound sensors, and the apparatus is manually moved in use to different positions on the subject's chest to (sequentially) acquire sound signals at the different receive locations across the chest. This set of signals corresponding to the different receive locations is then received at the controller which processes the signals as described above.

In a second main set of embodiments, an arrangement comprising a plurality of sensors is provided. The sensors are fixed in position on the apparatus. The sensors are arranged such that, when the apparatus is in place on a subject's chest, each sensor is positioned at a different location across the chest of the subject. Each can therefore receive sound signals at a different location across the chest.

More particularly, in examples of this set of embodiments, the arrangement may comprise a plurality of subsets of sound sensors, each subset for sensing in use sound signals at a different receive location on the chest of the subject;
and the controller is adapted to process the sound signals received at the arrangement of sound sensors, and to classify each of the subsets of sound sensors as either rib-aligned or intercostal space-aligned, based on an intensity of sound signals received at each of the subsets.

Here, the apparatus is arranged such that when in place on the subject's chest, the arrangement of sound sensors is spread across the patient's chest, and in particular across a plurality of the subject's ribs. Some of the subsets of sound sensors will be positioned above ribs of the subject. Some of the subsets of the sound sensors will be positioned above intercostal spaces (gaps between ribs). The controller analyzes sound signals from each subset of sensors to detect if it is above a rib or above a space.

In either of the two main sets of embodiments, rib-aligned means aligned with a rib of the subject; intercostal space aligned means aligned with an intercostal space (between two neighboring ribs).

In some examples, the arrangement may comprise rows and columns, and wherein the subsets are individual rows or columns of the arrangement. The rows may be straight lines or may be curved lines.

For example, the arrangement may be an array of sound sensors, comprising a plurality of rows, and wherein the subsets of sound sensors are individual rows of said array, and
wherein the controller is adapted to process the sound signals received at the array of sound sensors, and to classify each row of the array as either rib-aligned or intercostal space-aligned, based on an intensity of sound signals received at each row.

The array may be a regular or non-regular arrangement of sensors.

The subsets of sound sensors may be individual rows of such an array, or may be subsets or portions of individual rows in other examples.

In more detail, in these embodiments, there is provided an acoustic sensing apparatus for sensing rib positions of a subject, the apparatus for placement in use on a chest of the subject, the apparatus comprising:
an array of sound sensors adapted to sense sound signals from inside the chest of the subject, the array comprising a plurality of rows, and
a controller adapted to:
   process the sound signals received at the array of sound sensors, and to classify each row of the array as either rib-aligned or intercostal space-aligned, based on an intensity of sound signals received at each row.

The controller may be operable to implement a localized, e.g. directional, sound pick-up using the arrangement of sound sensors. The controller may be adapted to localize the sound pick-up based on the classifications of each of the subsets of the arrangement.

In this case, the apparatus can be controlled to provide localized or directional sound sensitivity, and where the direction of the sound sensitivity can be adjusted, and wherein the direction may be adjusted based on the arrangement subset classifications, e.g. the array row classifications.

Directional sound sensing using an arrangement of sound sensors can be performed in different ways.

In simple examples, it may comprise deactivating sound sensors which are classified as rib-aligned, and/or activating sound sensors which are classified as intercostal-space aligned.

Additionally or alternatively, it may comprise a process of compiling signals from the individual sound sensors with a defined pattern of delays implemented between them. This leads to a directional aperture selection, wherein sounds from a select spatial region only are extracted from the sound signals of the arrangement of sensors. This is analogous to beam steering the ultrasound domain. This will be described in more detail in the following section.

In other examples, physical steering of one or more sound sensors may be performed.

The manner in which sound intensity is assessed to determine the classifications may depend upon a mode of operation, and in particular may depend upon the originating location of the sounds which are sensed.

In at least one set of embodiments, the one or more sounds sensors are configured to sense sounds generated inside the body (i.e. organic, bodily sounds). The apparatus may for example be configured for sensing and processing heart-generated sound signals, lung generated sound signals, breathing sounds, and/or for sensing and processing subject vocal sounds.

In this case, the controller may be adapted to classify a location, e.g. a subset, as rib-aligned based on sensing a relative lower intensity sound signal, and to classify a location, e.g. a subset, as intercostal space-aligned based on sensing a relative higher-intensity sound signal.

The rib bones intercept and attenuate sounds travelling from inside the body, leading to lower intensity pick-up at sensors positioned above ribs, than for sensors positioned above intercostal spaces.

In these examples, the sensing apparatus may simply comprise a set of sound sensors, without comprising any sound generator. Hence, in this case, the apparatus may be configured to use intrinsic heart and/or breath generated sounds, such as heart beat or heart murmur, or inhalation sounds, to perform the position sensing.

Additionally or alternatively the apparatus may be configured for sensing and processing subject vocal sounds transmitting through the chest surface.

In at least a further set of embodiments, the apparatus may further comprise at least one sound generator adapted to generate sound signals for transmission into the chest, and wherein the sound sensors are adapted for sensing said generated sound signals.

In this embodiment, the apparatus includes a sound generator to generate stimulus signals for propagating into the chest. The sound sensors then sense the reflections or echoes of those signals received back from the chest. Here the sounds ultimately sensed originate from above the chest.

In this case, the controller may be adapted to classify a location, e.g. a subset of sound sensors, as rib-aligned based on sensing a relative higher intensity sound signal, and to classify a location, e.g. a subset, as intercostal space-aligned based on sensing a relative lower-intensity sound signal.

Bone conduction means that where a sound generator is used, signals received at sound sensors above ribs will in general have higher intensity than signals received at sound sensors aligned with an intercostal space.

In advantageous examples, the sound generator may be adapted to generate sound signals comprising a spectrum or band of multiple sound frequencies, for example a broad band signal comprising the 1-3 kHz frequency band. A broader band sound signal will reduce the effect of any possible resonances in the rib-cage at certain specific frequencies, which could lead to erroneous measurements. Measurement reliability is therefore improved.

Optionally, the apparatus may have multiple selectable modes, enabling operation with either artificially generated sounds (using a sound generator) or with intrinsic sounds (heart sounds, breath sounds and/or vocal sounds). The signal analysis and classification processes may be altered according to which mode is active.

The controller may be further adapted to generate a map of the subject's rib positions based on the derived categorizations of the receiving locations, for example the categorizations of the subsets of sound sensors of the arrangement.

The map may be a sound intensity map. The map is a spatial map. Generating the map may be based in part on a known (e.g. stored) spacing between sounds sensors of the arrangement. The map may be a visual map, or may take any other representative form of the spatial arrangement or structure of the ribs and intercostal spaces.

The controller may be adapted to label, e.g. number, the detected ribs and/or intercostal spaces. This information may be included as a part of a generated map of the rib positions.

The controller may be adapted to derive a signal intensity distribution for the sound signals across a spatial region sensed by the arrangement of sound sensors.

This means to determine a spatial distribution of sound intensity across a spatial area to which the arrangement is acoustically sensitive (with which the arrangement is in acoustic communication). This will typically correspond to a spatial region across the chest area of the subject. The controller may generate an intensity distribution map or plot.

The controller may be further adapted to identify one or more sound intensity hotspots within the signal intensity distribution.

Hotspots means regions of relative high signal intensity, for instance exceeding some pre-defined threshold.

These may be for example regions of the intensity distribution in which the sensed sound intensity is above a defined percentage threshold of a maximum sound intensity within the distribution.

A sound intensity hotspot is indicative of an anatomical object or regions of interest. In examples, based on the location of the hotspot relative to the detected rib positions, the anatomical object to which the hotspot corresponds can be determined.

Example regions or objects of interest which may be identified include: the heart mitral valve, the heart tricuspid valve, heart aortic valve, and the pulmonary artery. These are the four key areas for an auscultation procedure.

The sensing apparatus according to an advantageous set of embodiments may be further configured for performing auscultation of the subject's chest using the arrangement of sound sensors. The sensing apparatus may include control means for controlling automated auscultation of the subject's chest using the arrangement of sound sensors. The control means may comprise the central controller referred to above, or may comprise a further separate controller.

Additional components may be included for this purpose, or the apparatus may be operable to perform the auscultation using the same components discussed above.

According to one or more embodiments, responsive to the classification of the subsets of the arrangement, the controller may be adapted to activate the subsets of the arrangement which are classified as intercostal space-aligned for performing auscultation, and/or to deactivate the subsets of the arrangement classified as rib-aligned.

In this way, sound signals are sensed using only sensors which are in proper acoustic communication or alignment with the interior of the chest region, i.e. not via a rib, which would interfere with the received sound signals.

The controller may be adapted to extract from the sound signals of the arrangement of sound sensors a localized or directional sound signal corresponding to a location or direction of the one or more identified hotspots within the intensity distribution.

In this example, a localization of sound pick-up is directed to key areas of anatomical interest for auscultation. As explained above, sound hotspots are indicative of locations of such regions.

Extracting the localized sound signal may comprise compiling signals from a determined set of sensors within the array, with a particular pattern of delays imposed. This results in an effective steering of the sound sensing array. This may be performed using signals received only at the sensors which are within subsets classified as intercostal space-aligned.

According to one or more embodiments, the subsets of sound sensors in the array may be shaped so as to follow contours of the ribs of a typical human. For example, the arrangement may include a plurality of rows, the rows shaped to follow the contours of the ribs of a typical human being. In this way, the subsets, e.g. rows, may in use align with the ribs, so that some rows lie above a rib and some rows lie above an intercostal space.

In some examples, at least the arrangement of sound sensors may be integrated in a body-mountable patch, for mounting on the chest of the subject.

According to an advantageous set of embodiments, the sensing apparatus may be further configured for performing ultrasound examination, for example ultrasound imaging. In this case, the sensing apparatus may further comprise an ultrasound sensing arrangement comprising one or more ultrasound transducers.

The ultrasound transducers may be CMUT transducers.

The ultrasound sensing arrangement may comprise an array of ultrasound transducers. The sensing arrangement may include means for performing ultrasound beam steering using the array. The transducer array may be controlled to direct ultrasound signals toward one or more of the detected hotspots within the derived sound intensity distribution map, e.g. to steer though the intercostal spaces between the ribs.

Here, the detected locations of the hotspots are used to guide the ultrasound imaging. As noted above, hotspots are indicative of anatomical regions of interest.

Examples in accordance with a further aspect of the invention provide an acoustic sensing method for sensing positions of ribs within a chest of a subject, comprising:
receiving sound signals at a plurality of different locations across the chest of the subject using an arrangement of one or more sound sensors; and
processing the sound signals received at the plurality of different locations to classify each location as either rib-aligned or intercostal space-aligned, based on an intensity of sound signals received at the location.

Implementation options and details for each of the above steps may be understood and interpreted in accordance with the explanations and descriptions provided above for the apparatus aspect of the present invention (i.e. the sensing apparatus aspect).

Any of the examples, options or embodiment features or details described above in respect of the apparatus aspect of this invention (in respect of the sensing apparatus) may be applied or combined or incorporated mutatis mutandis into the present method aspect of the invention.

According to one or more sets of embodiments, the method may comprise
receiving sound signals at an arrangement of sound sensors, the arrangement placed on the chest of the subject, and the arrangement comprising a plurality of subsets, and
processing the sound signals received at the arrangement of sound sensors to classify each subset of the arrangement as either rib-aligned or intercostal space-aligned, based on an intensity of sound signals received at each subset.

The arrangement may comprise a plurality of rows, and wherein the subsets are individual rows of the arrangement, or portions of individual rows for instance.

The arrangement may comprise an array.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example sensing apparatus according to one or more embodiments;
Fig. 2 shows a sound intensity distribution plot of the chest area obtained using the apparatus of Fig. 1;
Fig. 3 shows a further example sensing apparatus according to one or more embodiments;
Fig. 4 shows a sound intensity distribution plot of the chest area obtained using the apparatus of Fig. 3;
Fig. 5 shows a sound intensity distribution map or plot obtained using an embodiment, the map showing sound hotspot locations;
Fig. 6 shows the corresponding anatomical features to which the hotspots of Fig. 5 correspond;
Fig. 7 illustrates an acoustic beamforming method for achieving directional sound pickup using an array of sound sensors;
Figs. 8 and 9 show a flow diagram illustrating an example workflow for one or more embodiments; and
Fig. 10 shows a block diagram of an example acoustic sensing method according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an acoustic sensing apparatus and method for acoustically surveying the chest area of a subject, and in particular the rib cage. The apparatus includes an arrangement of one or more sound sensors and a controller, wherein the arrangement is configured to be placed on the chest of the subject in use, and to detect acoustic signals received from inside the chest. Based on the signal intensities picked up at different locations across the chest using the arrangement of sensors, the different locations are each classified as being positioned either above a rib of the subject, or above an intercostal space of the subject.

In particular examples, the arrangement includes a plurality of subsets of sound sensors, and wherein each subset is for sensing in use sound signals at a different location across the subject's chest. The controller in this case may classify each subset of the arrangement based on intensities of sound signals received at the subset. The subsets may be individual rows of the arrangement for instance.

By detecting positions of each subset relative to the ribs, more precise directional signal pickup may in some embodiments be implemented. For example, based on a mapped position of the ribs relative to the different subsets of the array, a location of one or more anatomical features of interest may be estimated, and the device tuned for directional sound pick-up from these one or more locations. Ultrasound imaging components may also be included. Beam-steering of the ultrasound transducers may in some embodiments be guided based on the detected or mapped rib positions.

Particular embodiments of the invention may provide a heart monitoring tool for performing cardiac examination, comprising a microphone array (which may have a controllable directionality) and an ultrasound imaging means. Preferably both the microphone and the ultrasound functionalities may be spatially directed.

A remote cardiac examination may be performed using such a device.

As discussed above, two main implementation options are possible.

In one main set of embodiments, the apparatus is provided having just a single sound sensor, or a small patch of sound sensors, and the apparatus is manually moved in use to different positions on the subject's chest to (sequentially) acquire sound signals at the different receive locations across the chest. This set of signals corresponding to the different receive locations is then received at the controller which processes the signals as described above.

In a second main set of embodiments, an arrangement comprising a plurality of sensors is provided. The sensors are fixed in position. The sensors are arranged such that, when the apparatus is in place on a subject's chest, each sensor is positioned at a different location across the chest. Each can therefore receive sound signals at a different location across the chest.

More particularly, in examples of this set of embodiments, the arrangement may comprise a plurality of subsets of sound sensors, each subset for sensing in use sound signals at a different receive location on the chest of the subject;
and the controller is adapted to process the sound signals received at the arrangement of sound sensors, and to classify each of the subsets of sound sensors as either rib-aligned or intercostal space-aligned, based on an intensity of sound signals received at each of the subsets.

The arrangement may comprise an array having multiple rows, and wherein the subsets of the arrangement correspond to individual rows of the array. Below are described embodiments having such a configuration. However, it is to be understood that this is exemplary only, and the broad inventive concept extends to other arrangements with differently configured subsets of sound sensors. Hence, any use of the term 'array' or 'row' may be understood as replaceable with the term 'arrangement' and 'subset' without loss of advantageous effects of the embodiments.

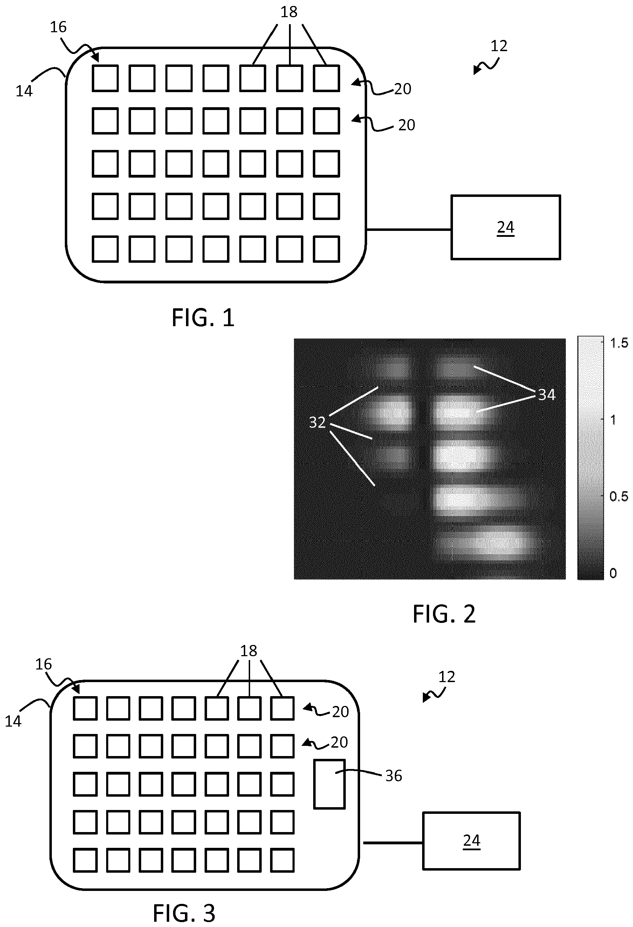

Fig. 1 shows an example sensing apparatus 12 according to one or more embodiments of the invention. The apparatus is for sensing rib positions of a subject. In use, the apparatus is placed on the chest of the subject.

The apparatus 12 comprises a two-dimensional array 16 of sound sensors 18 adapted to sense sound signals from inside the chest of the subject. The array comprises a plurality of rows 20. The rows are preferably shaped or contoured so as to follow the natural contours of the ribs of the subject. This means that, when in position, some of the rows will be aligned above a rib, and some of the rows aligned above an intercostal space between a rib.

The rows 20 may be linear rows in some examples.

The apparatus 12 further comprises a controller 24, operatively coupled with the array 16 of sound sensors 18.

The controller 24 is configured to process the sound signals received at the array 16 of sound sensors 18, and to classify each row 20 of the array as either rib-aligned or intercostal space-aligned, based on an intensity of sound signals received at each row.

Rib-aligned may mean that the row is positioned such that a rib of the subject is in an acoustic path between the row and the origin of the sound signals in the chest.

In the present example, the array 14 of sound sensors 18 is integrated in a body-mountable patch 14, for attaching to a chest of the subject. The patch may have an adhesive backing to allow it to be removably attached to the chest.

However, use of a patch is not essential. The array 16 may be provided mounted on or within any support structure. This may be a planar support structure, for instance comprising a PCB. The support structure is preferably flexible. A non-planar structure is also possible, for instance a housing comprising the sensors positioned across a skin-contact area at the base.

The sound sensors 18 may each comprise one or more acoustic transducer elements. The sound sensors may each comprise a microphone.

The sound sensors 18 may in some examples each comprise one or more ultrasound transducers, having a sensitive range which extends to cover ultrasound frequencies and also below ultrasound frequencies. Preferably the ultrasound transducer elements may be capacitive micromachined ultrasound transducer (CMUT) elements.

A CMUT element can be configured for detecting one or both of low frequencies (Hz-KHz ranges) and MHz ultrasonic frequencies.

This may be achieved in some examples by modifying the CMUT construction to render the element more susceptible to audio frequency vibrations. This may include for example increasing the mass of the vibrating CMUT membrane. This thereby lowers the membrane's resonant frequency, which leads to optimization of the element for sensing vibrations in the Hz-kHz frequency range.

Additional mass may be added to the CMUT membrane during manufacture based on sputter deposition of for instance a heavy metal layer such as tungsten or gold. This may be followed by patterning of the element, for instance using a litho mask, and an etching step.

In a second example, the area of the CMUT membrane may be modified to adjust the frequency sensitivity (increased for lower frequency sensitivity and vice versa).

In some examples, the membrane thickness may be varied to adjust the frequency sensitivity (greater thickness for sensitivity to lower frequencies and vice versa).

In a further example, the frequency sensitivity of the CMUT element can be adjusted during use, based on varying the bias voltage. For example, CMUT elements may be operated in collapse mode (by applying the bias voltage) to sense ultrasound frequencies, and operated in non-collapse mode (by removing the bias voltage) for sensing sub-ultrasound sound frequencies. Thus the same single set of CMUT elements may be selectively used both for ultrasound sensing (and/or transmission) and for sound sensing (although not at the same time).

Use of CMUT elements for the sound sensors 18 is however not essential. Any other form of sound sensor may be used, for instance conventional microphones.

In the case that ultrasound transducer elements are included, these may optionally also be used for performing ultrasound sensing and/or imaging in addition to sound sensing.

The intensity of the sound signals may be sensed based on detected signal strength, power, energy or any other intensity-related parameter.

The sound signals may be signals as a function of time, for instance representing sound intensity as a function of time.

Although the controller 24 in Fig. 1 is shown in the form of a unitary control unit, this is for illustration only. The functions performed by the controller, both in the present embodiment, and in any embodiments described throughout this disclosure, may alternatively be performed by a more distributed set of components, for instance multiple control elements or processors, or by processing elements included in one or more of the other components of the sensing apparatus 12.

In use, the patch 14 comprising the array 16 of sound sensors 18 is placed on the subject's chest, extending over their rib cage. The array of sensors is adapted to sense sounds via the chest wall, and the controller 24 receives and analyzes these signals.

Heart and/or breath sounds may in some examples be picked up by the sensors 18 of the array 16 and used in performing the position analysis. The apparatus is in this case configured to use intrinsic heart and/or breath generated sounds, such as the subject's heart beat or heart murmur, or respiration sounds, to perform the position sensing.

In this example, the apparatus may be configured for sensing sounds in a characteristic frequency range for the heart and/or breath sounds, or a frequency filter applied by the controller to extract such frequencies. The range may be empirically derived for a given patient, or typical values may be pre-programmed in the controller, or used for designing the intrinsic sensor sensitivities.

Additionally, or alternatively the apparatus may be configured for sensing and processing subject vocal sounds (subject vocalizations). These may be arbitrary vocal sounds, or a pre-determined speech pattern which the subject may be prompted to perform. Vocal resonance of the vocal sounds generated by the patient may in this example be used to perform the position sensing.

Based on analysis of the intensities of the received sound signals at the array 16 of sound sensors 18, the controller 24 may estimate which sound sensors 18 are positioned above ribs of the subject and which are above intercostal spaces.

Optionally, with this information contours of the ribs may be determined and traced. A map of the (anterior) rib cage may optionally further be constructed. This may be a spatial map. The map may indicate positions of the ribs. It may include labels, e.g. numbers, for the ribs and/or intercostal spaces.

The controller may be adapted to classify a row as rib-aligned based on sensing a relative higher lower sound signal, and to classify a row as intercostal space-aligned based on sensing a relative higher-intensity sound signal.

The rib bones intercept and attenuate sounds travelling from inside the body, leading to lower intensity pick-up at sensors positioned above ribs, than for sensors positioned above intercostal spaces.

Here, the controller is adapted to associate rib-alignment with a relative lower intensity sound signal, and is adapted to associate intercostal space alignment with a relative higher-intensity sound signal.

Hence, rows at which relative lower intensity signals are received are classified as rib-aligned, and rows at which relative lower intensity signals are received are classified as intercostal space-aligned.

Note that this classification mode is applicable for embodiments in which sensed sound signals originate from inside the body, e.g. heart or breath sounds. In embodiments to be described below, in which an artificial sound generator is used, the applied intensity analysis is different due to the effects of bone conduction. This will be explained below.

An example signal intensity map or plot obtained using the array of sound sensors, in position on the chest of a subject, is shown in Fig. 2. A key is shown on the right-hand side, with the units corresponding to sound intensity normalized to the source intensity of the sound signals. Lighter shades show higher intensity.

The contours and outlines of the ribs, and the intercostal spaces, can be seen in the plot. The indicated lower intensity areas 32 correspond to the locations of the ribs. The indicated higher intensity regions 34 correspond to the locations of the intercostal spaces.

In use, the controller analyzes the sound signals received at the array of sound sensors to determine which rows of the array are rib-aligned and which are intercostal space aligned. An example processing workflow for deriving the classifications of the rows, based on signals received at the embodiment of Fig. 1 is outlined below.

First, the input signals at each sound sensor are pre-processed with a low-pass filter. The filter extracts only frequency ranges corresponding to the intended pickup target (e.g. heart murmur). For example, the low pass filter may have a 600Hz maximum cut-off for extracting only heart murmur sounds. This is just one example of a cut off frequency, and other specific values may be used in other examples.

After pre-processing, categorization of the rows of the array may be performed. Optionally, each and every sound sensor may be categorized as either rib aligned or intercostal space aligned. Based on this, each row of sound sensors may be categorized.

To perform the categorization, first a (moving window) average may be applied on the sound signals from the sound sensors to identify a mean local intensity map.

Following this, the sound sensors at which the received signal has an intensity of less than 50% of the highest mean intensity derived above are categorized as rib-aligned. All other sound sensors may be categorized as being positioned between two ribs (at an intercostal space).

The threshold of 50% represents just one example. In other examples, a different specific value may be used. The threshold may in some examples be determined based on analysis of a training dataset.

Where subject vocalization sounds are to be used for the position detection, the workflow may be slightly modified.

The workflow may optionally first comprise performing a calibration step, in which the subject is prompted to generate a pre-determined, or arbitrary, vocalization pattern. During vocalization, signal intensities are measured at each sound sensor in the array, at both a high frequency (e.g. 1024Hz) and a low frequency (e.g. 128 Hz). Alternatively, a Fourier transform into the frequency domain may be applied to the intensity signals received at each sound sensor in the array, and the intensities for the high and low frequencies identified from this.

The numerical values cited above are exemplary only, and other values may alternatively be used.

A ratio of the measured intensities at the low and the high frequency is then calculated for each sound sensor.

Following this optional calibration procedure, rib-detection may be performed. The subject is prompted to again generate vocal sounds, and the sound intensities at each sensor measured for at least each of the low and high frequencies mentioned above.

For sound sensors where the low frequency power is a minimum defined threshold above the high frequency power, the sensors may be categorized as rib-aligned. Other sensors are categorized as intercostal space aligned.

The minimum threshold may be a power of at least 3 dB higher than the high frequency power, e.g. the low frequency power is 2 times higher than the high frequency power. These values are exemplary only, and other specific values can be used.

According to further embodiments, instead of using intrinsic heart, breath or vocal sounds to perform the position detection, a sound signal may be artificially generated using a provided sound generator.

An example of such an embodiment is shown in Fig. 3.

The sensing apparatus 12 of the example of Fig. 3 is the same as the example of Fig. 1 except for the additional provision of a sound generator 36 adapted to generate sound signals for transmission into the subject's chest. The sound generator 36 may be a microphone for example.

The sound sensors 18 in the array are adapted for sensing the sound signals generated by the sound generator 32.

In use, the controller 24 analyzes the sound signals received at the array 16 of sound sensors 18 to determine which rows of the array are rib-aligned and which are intercostal space aligned. An example processing workflow for deriving the classifications of the rows, based on signals received is outlined below.

First, the sound generator 36 is controlled to generate an output sound signal of a selected frequency (e.g. 1 kHz) or frequency range. Due to the placement of the apparatus on the chest of the patient, with the sound generator applied against the chest wall, the generated sound signal is transmitted from the speaker into the chest. Preferably, the frequency of the generated sound signal is at least 1 KHz, but may be any frequency, e.g. up to 20KHz.

In preferred embodiments, a broader band sound signal (e.g. 1-3 kHz) may be generated, as this will reduce the effect of any possible resonances in the rib-cage at certain specific frequencies, which could lead to erroneous measurements.

The received signals at each sound sensor 18 are then pre-processed with a frequency filter. The filter extracts only frequency ranges corresponding to the intended pickup target (i.e. the output sound signals of the sound generator 36). For example, for a 1 kHz frequency generated signal a Band Pass Filter with center frequency of 1 kHz and a 50% Band Width would be suitable. This is just one example of a cut off range, and other specific values may be used in other examples.

After pre-processing, categorization of the rows of the array as rib aligned or intercostal space aligned may be performed. Optionally, each and every sound sensor may be categorized as either rib aligned or intercostal space aligned. Based on this, each row of sound sensors may be categorized.

To perform the categorization, first a (moving window) average may be applied on the sound signals from the sound sensors to identify a mean local intensity map.

Following this, the sound sensors at which the received signal has an intensity of greater than 50% of the highest mean intensity derived above are categorized as rib-aligned.

All other sound sensors may be categorized as being positioned between two ribs (at an intercostal space).

The threshold of 50% represents just one example. In other examples, a different specific value may be used. The threshold may in some examples be determined based on analysis of a training dataset.

Hence, in this embodiment, in which a sound generator is used, the controller is adapted to classify a sound sensor as rib-aligned based on sensing a relative higher intensity sound signal, and to classify a sound sensor as intercostal space-aligned based on sensing a relative lower-intensity sound signal.

This is because the sound signals originate from above the chest, rather than below it, which leads to bone conduction effects. Bone conduction means that signals received at sound sensors above ribs will have higher intensity than signals received at sound sensors aligned with an intercostal space.

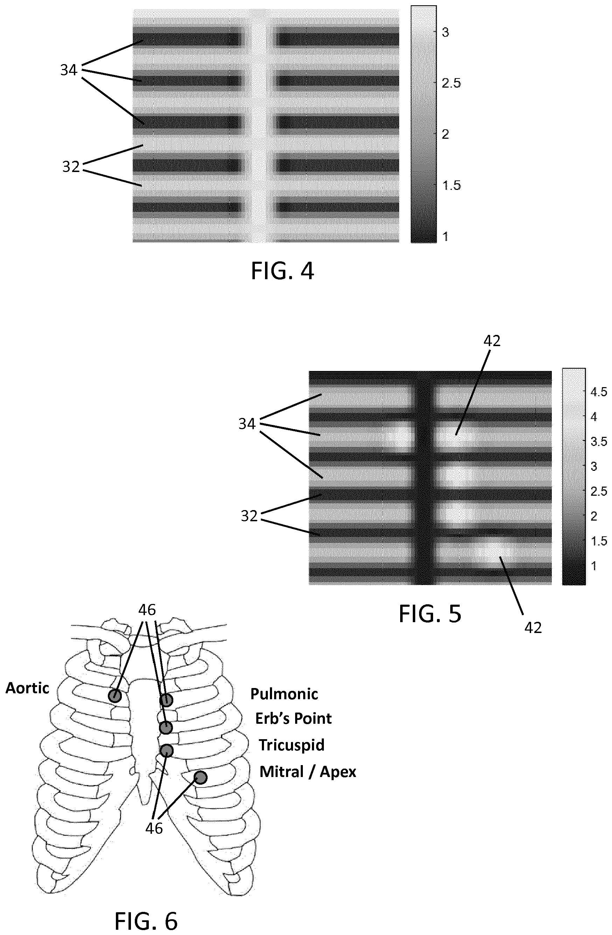

An example signal intensity map or plot obtained using the array 16 of sound sensors 18 in combination with the sound generator 36, in position on the chest of a subject, is shown in Fig. 4. A key is shown on the right-hand side, with the units corresponding to sound intensity normalized to the source intensity of the sound signals. Lighter shades show higher intensity.

The contours and outlines of the ribs, and the intercostal spaces, can be seen in the plot. The indicated higher intensity areas 32 correspond to the locations of the ribs. The indicated lower intensity regions 34 correspond to the locations of the intercostal spaces.

In accordance with any of the above embodiments, the sensing apparatus may be further configured for performing auscultation of the subject's chest using the array of sound sensors.

In accordance with any of the above embodiments, in some examples, responsive to the classification of the rows of the array, the controller 24 may be adapted to activate the rows of the array which are classified as intercostal space-aligned for performing auscultation, and/or to deactivate the rows of the array classified as rib-aligned.

In this way, sound signals are sensed using only sensors which are in proper acoustic communication or alignment with the interior of the chest region, i.e. not via a rib, which would interfere with the received sound signals.

In accordance with any of the embodiments described above, the controller may be adapted to derive from the sound signals received at the array of sensors, a signal intensity distribution for the sound signals across a spatial region to which the array is sensitive.

This means to determine a spatial distribution of sound intensity across a spatial area to which the array is acoustically sensitive (with which the array is in acoustic communication). This will typically correspond to a spatial region across the chest area of the subject. The controller may generate an intensity distribution map or plot.

In simple examples, the signal intensity distribution may comprise simply a single sound intensity value or signal for each of the sensors in the array. Alternatively, a more continuous map or plot of intensity distribution may be generated across the spatial area to which the array is sensitive, e.g. a chest area.

In accordance with any of the embodiments described above, based on the categorization of the rows of the array, the controller 24 may be adapted to generate a map of the positions of the ribs. This may be a spatial map. It may represent contours or outlines of the ribs and the intercostal spaces. It may also include a plot or representation of measured sound signal intensity over the chest area.

Optionally, the detected ribs may be numbered. Labels may be added to relevant ribs in the map indicating a number.

The numbering may be performed using a connected component analysis. This may require use of a generated signal intensity distribution, as discussed above. A connected component analysis comprises processing the pixels of a derived signal intensity distribution map, and identifying connected pixels and non-connected pixels. This is done based on detecting steep changes in intensity values from one pixel to the next (disconnected pixels) and vice versa. Using this procedure, performed row by row, the start and end points of each rib, and each intercostal space can be mapped, and each of the ribs numbered.

The intercostal spaces may also be numbered using connected component analysis.

An example workflow for a connected component analysis is set out below. A first pass of the workflow comprises the following steps.
I. Iterate through each pixel of a derived 2D signal intensity map by row, then by column (Raster Scanning)
II. If a pixel is located within an intercostal space. (as detected by one of the rib detection procedures described above)
   a. Identify the neighboring pixels of the current pixel. For this, four-connectivity may be used, in which case the neighbors are the pixels immediately north, south, east and west of the current pixel. Alternatively, eight-connectivity can be used, in which case neighbors include also north-east, north-west, south-east, south-west pixels.
   b. Identify the neighbor (of the four or eight) with the smallest number label and assign it to the current pixel
   c. If there are no neighbors, uniquely label the current pixel and continue
III. Store the equivalence between neighboring labels

A subsequent (second) pass then comprises the following steps:
I. Iterate through each pixel of the data by row, then by column
II. If the pixel is not located within an intercostal space
   a. Relabel the pixel with the lowest equivalent label

This example procedure is described in more detail in the paper: Lifeng He; Yuyan Chao,; Suzuki, K. (1 May 2008). "A Run-Based Two-Scan Labeling Algorithm". IEEE Transactions on Image Processing. 17 (5): 749-756. Connected component analysis as a general approach will be well known and understood by the skilled person.

The controller 24 may be further adapted to identify one or more sound intensity hotspots within the signal intensity distribution.

Hotspots means regions of relative high signal intensity, for instance exceeding some pre-defined threshold.

These may for example be regions of the intensity distribution in which the sensed sound intensity is above a defined percentage threshold of a maximum sound intensity within the distribution.

The intensity hotspots may correspond to regions within the spatial distribution meeting or exceeding a pre-defined intensity threshold. The threshold may by way of example be set at a value between 70%-80% of the maximum intensity detected in the intensity distribution, for example 75%. This is one example value only and other values for the threshold may alternatively be used.

The applied threshold for detection of a hotspot may optionally be set based on a derived histogram of signal intensity over the sensed area, i.e. a defined percentage of a maximum intensity value in the histogram.

A sound intensity hotspot is typically indicative of an anatomical object or region of interest. In examples, based on the location of the hotspot relative to the detected rib positions, the anatomical object to which the hotspot corresponds can be determined.

Example regions or objects of interest which may be identified include: the heart mitral valve, the heart tricuspid valve, heart aortic valve, and the pulmonary artery. These are the four key areas for an auscultation procedure.

The anatomical location to which a hotspot corresponds may be detected based on one or more of: which side of the chest (right/left) the hotspot is located on, the number of the intercostal space at which it is found (these numbered consecutively from top to bottom), and the intensity of the signal in the area of the detected hotspot.

For example, the aortic and pulmonary auscultation positions are located at the second right-hand intercostal space, at the right sternal border and the second left-hand intercostal space, left sternal border respectively. The mitral and tricuspid auscultation positions are respectively found at the fifth intercostal space, left mid-clavicular line at the and fourth left intercostal space, left sternal border.

This is illustrated in Fig. 5 which shows an example signal intensity map for the chest area. The ribs are identifiable in the map as relative low intensity regions 32, and the intercostal spaces identifiable as relative high intensity regions 34. Lighter shades indicate higher intensities.

Various intensity hotspots 42 are visible within the intercostal spaces 34.

Fig. 6 illustrates the corresponding anatomical positions to which each of the detected intensity hotspots corresponds. Each corresponds to one of the four main auscultation locations discussed above: the mitral valve, the tricuspid valve, aortic valve, and the pulmonary artery.

In some examples, an acoustic anatomical model may be accessed by the controller and used to assist in identifying the anatomical location to which each hotspot corresponds.

The identified hotspots may be used to directionally tune the array of sound sensors 18 to receive in the direction of the hotspots.

For example, the controller 24 may be adapted to extract from the sound signals of the array 16 of sound sensors 18 a directional sound signal corresponding to a direction of the one or more identified hotspots 42 within the intensity distribution.

In this example, a directionality of sound pick-up is directed to key areas of anatomical interest for auscultation. As explained above, sound hotspots are indicative of locations of such regions.

Extracting the directional sound signal may comprise compiling signals from a determined subset of sensors within the array, with a particular pattern of delays imposed. This results in an effective steering of the sound sensing array. This may be performed using signals received only at the sensors which are within rows classified as intercostal space-aligned.

In this example, the steering and focusing of the sound sensor array may be achieved in a similar way to that used for ultrasound beamforming; in particular, by use of a delay-and-sum (DAS) beamforming applied to the received acoustic signals of the array of sound sensors.

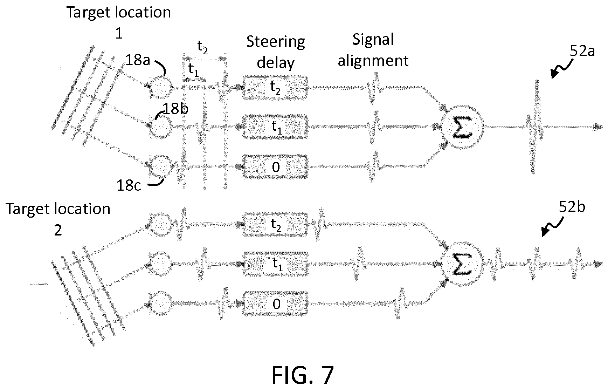

This is illustrated in Fig. 7 which shows how signals received at an example ordered row of sound sensors 18a, 18b, 18c, may be compiled together with different patterns of signal delays imposed in order to generate output composite signals 52a, 52b corresponding to different spatial directions. In the upper image, a first set of time delays is applied between the compiled signals, leading to an effective directional focus toward target source location 1. In the lower image, a second set of time delays is applied between the compiled signals, leading to an effective focus in target source location 2.

Steering of the sound pickup is particularly useful for instance in embodiments in which the sensing apparatus is further configured for performing auscultation of the subject's chest using the array of sound sensors.

In this set of embodiments, the apparatus may comprise a dedicated auscultation controller or processor for controlling the apparatus to perform an auscultation procedure, and optionally for processing received sound signals.

By extracting a signal corresponding to the select localized positions of the hotspots, a sound signal corresponding to the key anatomical auscultation positions may be extracted and analyzed. Hence, the sound sensors may be tuned to receive in the direction of the key anatomical locations discussed above.

In accordance with any of the above described embodiments, the sensing apparatus may further comprise means for performing ultrasound sensing (for instance ultrasound imaging), the means including one or more ultrasound transducer elements.

Preferably, the ultrasound transducer elements are CMUT transducer elements, although other varieties of ultrasound transducer may alternatively by used (e.g. piezoelectric).

In some examples, the ultrasound sensing and the sound sensing may be performed by the same array 16 of transducer elements. Here for instance transducer elements capable of operating both at ultrasonic frequency and at sub-ultrasonic frequency may be used. These same elements can be used to facilitate the sound sensors 18 for position analysis and to perform ultrasound sensing, e.g. ultrasound imaging. Example means for achieving this using CMUT transducer elements was discussed above.

In other examples, a separate set of ultrasound transducer elements and sound sensing elements may be provided. The two sets of elements may form part of a common array formation. For example, each row 20 of the array 16 described above may include both sound sensor elements and ultrasound transducer elements.

The ultrasound sensing means enables for instance ultrasound imaging to be performed using the sensing apparatus 12. The position analysis performed by the controller 24 may be used to guide the ultrasound imaging.

For example, in simple examples, the ultrasonic sensitive elements placed directly above the rib cage may be controlled not to activate, either in transmit or in receive beamforming mode, while those positioned above intercostal spaces are selectively activated. This helps in conserving power, since ultrasound elements blocked by the ribs are not activated.

In some examples, the ultrasound sensing arrangement may comprise an array of ultrasound transducers, and may include means for performing ultrasound beam steering using the array, and wherein the transducer array is controlled to direct ultrasound signals toward one or more of the detected hotspots. The ultrasound beam may be steering between the detected ribs, through the detected intercostal spaces. The generated map of the rib positions and/or generated sound intensity plot may be used to facilitate this.

The apparatus may comprise a beam steering control element to facilitate control of the beam steering.

The apparatus may include means for analyzing the collected ultrasound data to generate one or more ultrasound images.

According to one or more embodiments, the ultrasound sensing means may be used to detect a location of an anatomical object or region of interest within the subject's chest region. This location detection may then be used to guide localization of the sound sensing to the location of the detected anatomical object or region of interest, in accordance with the procedure described above.

For example, the arrangement of sound sensors may be used to detect the positions of the ribs, and based on this position detection, sound sensors detected to be located above ribs may be deactivated and sound sensors above intercostal spaces activated (or kept active). Following this, the ultrasound sensing means, for an instance an array of ultrasound transducers, may be controlled to capture ultrasound data representative of the chest region, e.g. ultrasound image data, and to apply data analysis to detect an anatomical object or region of interest, e.g. the mitral valve.

Localization or steering of the sound sensing toward the identified region of interest may then be implemented (using the remaining activated sound sensors) as described above, for instance based on appropriate processing of the sound signals received at the array of sound sensors.

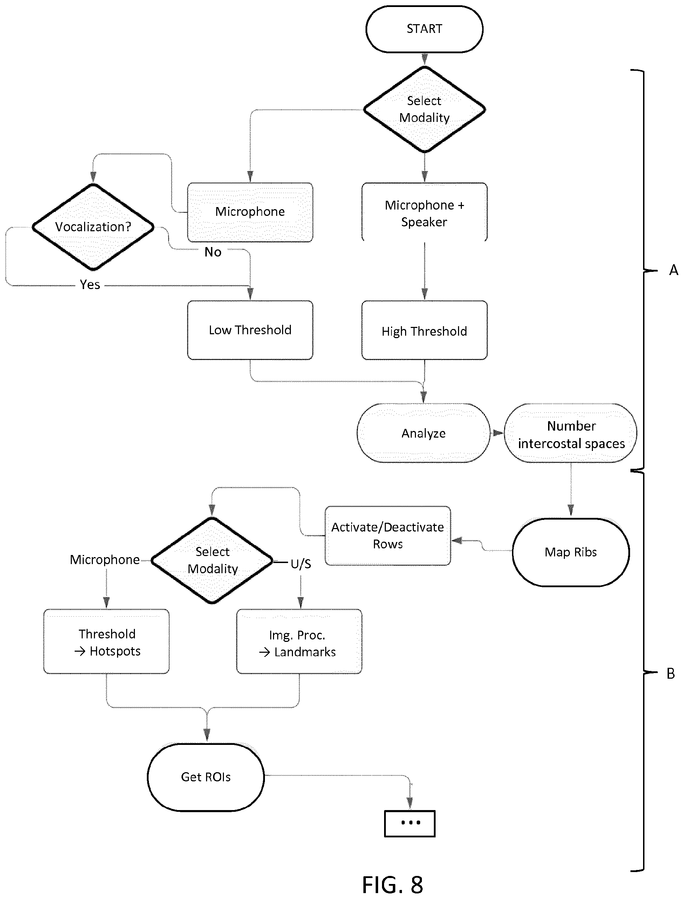

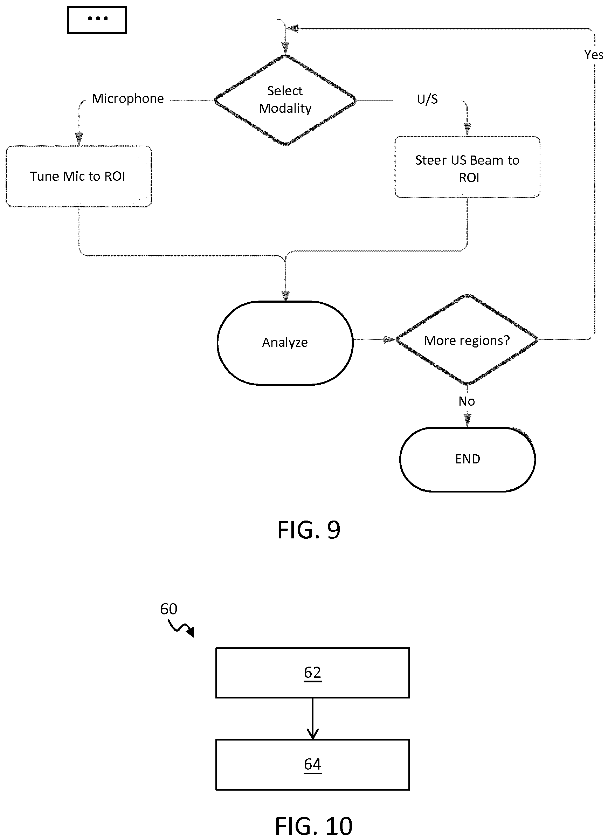

By way of summary of the various implementation options possible in different examples of the present invention, Fig. 8 and Fig. 9 show a flow diagram which outlines a workflow according to various possible embodiments. Fig. 8 outlines steps corresponding to the detection of rib positions, and also identification of local anatomical locations based on hotspot detection. Fig. 9 outlines steps corresponding to subsequent cardiac examination steps based on the identified anatomical locations, using either ultrasound imaging or acoustic auscultation.

Beginning with Fig. 8, the steps of section A relate to detecting and mapping the rib positions of the subject. The steps of section B relate to identifying hotspots within a generated intensity distribution and associating these with corresponding anatomical locations.

Section A comprises first selecting the modality to use for performing the position detection: either using an array of sound sensors (microphone) alone, and using heart sounds, breath sounds, and/or vocal sounds as the target sound signal, or using an array of sound sensors in combination with a dedicated sound generator (speaker). Each of these options is discussed in detail above.

For the microphone array alone, vocalization sounds may or may not be used. In either case, the intensity signals received at each row of the array of sound sensors are processed, and those rows at which the signals fall below a defined low threshold for intensity are classified as rib-aligned. Other rows are classified as intercostal space aligned.

Where the microphone array is used with a sound generator, instead, the array of sound sensors are processed, and those rows at which the signals fall above a defined high threshold for intensity are classified as rib-aligned. Other rows are classified as intercostal space aligned.

A signal intensity map or plot may be generated.

The signals from the array or across the intensity plot may be analyzed and the identified intercostal spaces numbered consecutively from top to bottom. This may be based on a connected component analysis (analyzing connected pixels within the intensity map). An example of this procedure was outlined in detail above.

Section B comprises using the detected rib and intercostal space positions to generate a map of the ribs. Following this, the controller deactivates those rows in the array classified as rib-aligned and/or activates those classified as intercostal space aligned.

Following this, section B comprises detecting specific anatomical locations within the generated intensity map. These locations may subsequently be used for directionally focusing acoustic or ultrasound signals for cardiac examination.

Detecting anatomical locations can be done either acoustically using the array of sound sensors (microphones), or using ultrasound ("U/S"). Where the sound sensors are used, hotspots are detected in the sound signal intensity map by identifying regions having an intensity falling above a defined minimum percentage of a maximum detected intensity within the whole distribution. This process was described in detail above. Where ultrasound is used, image processing may be used, including for instance use of segmentation or other image analysis techniques, and key landmarks in the scanner region detected.

Based on the detected acoustic signal hotspots or the ultrasound detected landmarks, the anatomical region of interest (ROI) corresponding to these detected points are determined.

Fig. 9 shows steps for performing cardiac examination based on steering or focusing of generated signals onto the identified ROIs. The cardiac examination may employ different modalities: either sound sensors (microphones) for performing auscultation, or ultrasound transducers, for performing ultrasound imaging. Where auscultation is performed, the array of sound sensors is directionally tuned or focused toward the identified ROIs. A procedure for achieving directional sound pickup was described above with reference to Fig. 7. Where ultrasound ("U/S") is used, beamforming may be used to steer the ultrasound beam toward the identified ROIs.

The obtained examination results may then be analyzed, and the procedure continued until all ROIs have been examined or imaged.

According to one or more embodiments, a map of the rib and/or intercostal space positions may be generated, as described in embodiments above, and local anatomical regions of interest (ROIs) further identified, as described above. These may be detected for instance by detecting hotspots in an acoustic intensity map.

According to one set of embodiments, the generated rib position map and detected ROI locations may be communicated to an operatively coupled virtual reality or augmented reality headset device, and a visual representation of the map and ROI positions generated on a display area of the device. The headset may be worn by a user for instance.

The headset may comprise a semi-transparent visor or glasses unit to be worn by a user. This headset may be configured to superpose the generated rib position map and ROI locations onto the chest of the subject as the user looks through the headset at the patient's chest area (i.e. augmented reality). This overlaid chest map may be used to assist ultrasound probe placement for an accurate measurement.

The headset might be used by an inexperienced user with little skill in correct placement of the apparatus. The headset might be worn by the subject for use in assisting them to position the sensing apparatus on their own chest. A doctor or other clinician could then remotely control the accurately positioned apparatus to performed a desired examination or set of examinations. The doctor might for example remotely choose the ROIs to examine and/or the modality (auscultation or ultrasound) to use, and analyze obtained results.

By way of further summary, Table 1 below sets out a variety of different implementation options, in accordance with the various embodiments discussed above.

**Table 1**

| **Rib Detection Modality** | **Technique** | **Principle** | **Outcome** |
|---|---|---|---|
| Using Sound Sensors alone | Sounds picked up from the chest wall through a microphone array are used to generate intensity maps | Sound intensity varies over chest wall area depending upon whether it is measured above a rib or intercostal space. | Intensity maps |
| | | | • This can be used to distinguish ribs and intercostal spaces |
| | | | • This can be also used to identify anatomical areas (e.g. areas of heart) |
| | | At certain areas over the chest wall (2 and 5^{th} intercostal spaces on Left and 2n^{d} space on Right), heart sounds are very loud due to proximity to underlying heart valves | |
| Using sound sensors and speaker | The speaker is positioned in the midline of the chest | As the sternum is connected with ribs the sound waves will travel through sternum into the ribs | Intensity maps |
| | | | • This can be used to distinguish ribs and intercostal spaces |
| | A test sound is transmitted via speaker(s) placed over sternum and the microphone array patch(es) placed over the chest surface begins receiving vibrations | | |
| | | The intensity of sounds recorded over ribs would be louder than over the intercostal space | |
| | The speaker sound is distinguished from heart sound using frequency and or intensity threshold | | |
| | An intensity map is generated | | |
| Using vocal resonance and sound sensors | Patient is asked to say a few specific words | The intensity of sounds recorded over intercostal spaces are be louder in this case than over the ribs | Intensity maps |
| | | | • This can be used to distinguish ribs and intercostal spaces |
| | Sounds picked up from the chest wall through a microphone array are used to generate intensity maps | | |

Table 2 shows different modality modes for performing cardiac examination of the subject, and the various control options for guiding steering of the modalities.

**Table 2**

| **Cardiac Examination Modality** | **Rib Detection Modality** | **Principle** |
|---|---|---|
| Sound sensors | Sound sensors | Rib cage and anatomical areas detected using sound sensors. |
| Sound sensors | Speaker + Sound sensors | Rib cage detected using microphone + Speaker. |
| Sound sensors | Sound sensors (Vocal resonance) | By rib cage detection using vocal resonance as the target sound. |
| Ultrasound | Sound sensors | Rib cage and anatomical areas detected using microphones. |
| Ultrasound | Speaker + Sound sensors | Rib cage detected using microphone + Speaker. |
| Ultrasound | Sound sensors (Vocal resonance) | Rib cage detection using vocal resonance as the target sound. |

Various embodiments have been described above comprising an arrangement of sound sensors having a plurality of subsets of sensors which are categorized as rib aligned or intercostal space aligned.

According to a second main set of embodiments of the invention, the apparatus may comprise an arrangement of only a single sound sensor, or a small patch of sound sensors, and the apparatus adapted to be manually moved in use to different positions on the subject's chest to acquire sound signals at the different receive locations across the chest. Hence here, sound signals are recorded at a plurality of different locations, but are captured sequentially using a single moveable sensor or sensor array, each signal captured by the same sensor or sensor array. This set of sequentially acquired signals, corresponding to the different receive locations, is then received at the controller. The controller processes the signals in accordance with one or more of the processing procedures described above to categorize each of the different locations as being rib aligned or intercostal space aligned based on an intensity of the sound signals received at the respective location.

Such a series of sequential sound measurements may be reconstructed to provide a detection of rib positions equivalent to that which may be achieved with an array of sound sensors. If enough sound measurements, e.g. random measurements, are made across the subject's chest, a good mapping of the ribs can be made. Following this, localization of sound sensing may also be performed in a quasi -sequential manner. For example, sound sensing could be performed in all sensing directions at all locations, the signals stored, and then based on the derived mapping of the rib positions, only the signals corresponding to the desired steering or localization direction within the chest retained. Others may be discarded.

Examples in accordance with a further aspect of the invention provide an acoustic sensing method for sensing positions of ribs within a chest of a subject.

An example acoustic sensing method 60 is shown in block diagram form in Fig. 10.

The method 60 comprises receiving 62 sound signals at a plurality of different locations across the chest of the subject using an arrangement 16 of one or more sound sensors 18.

The method 60 further comprises processing 64 the sound signals received at the plurality of different locations to classify each location as either rib-aligned or intercostal space-aligned, based on an intensity of sound signals received at the location.

In particular examples, the method may comprise receiving sound signals at an arrangement of sound sensors, the arrangement placed on the chest of the subject, and the arrangement comprising a plurality of subsets, and
processing the sound signals received at the arrangement of sound sensors to classify each subset of the arrangement as either rib-aligned or intercostal space-aligned, based on an intensity of sound signals received at each subset.

The arrangement may comprise a plurality of rows and wherein the subsets are individual rows of the arrangement, or portions of individual rows for instance.

Implementation options and details for each of the above steps may be understood and interpreted in accordance with the explanations and descriptions provided above for the apparatus aspect of the present invention (i.e. the sensing apparatus aspect).

Any of the examples, options or embodiment features or details described above in respect of the apparatus aspect of this invention (in respect of the sensing apparatus) may be applied or combined or incorporated mutatis mutandis into the present method aspect of the invention.

As discussed above, embodiments make use of a controller 24. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An acoustic sensing apparatus (12) for sensing rib positions of a subject, the apparatus for placement in use on a chest of the subject, the apparatus comprising:
an arrangement (16) of one or more sound sensors for sensing in use sound signals from the chest of the subject, and
a controller (24) adapted to:
process sound signals received in use at the arrangement of one or more sound sensors at a plurality of different receiving locations across the chest of the subject, and to classify each of the different receiving locations as either rib-aligned or intercostal space-aligned, based on an intensity of sound signals received at each of the locations.

2. An acoustic sensing apparatus as claimed in claim 1, wherein
the arrangement (16) comprises a plurality of subsets (20) of sound sensors, each subset for sensing in use sound signals at a different receiving location on the chest of the subject;
and wherein the controller is adapted to process the sound signals received at the arrangement of sound sensors, and to classify each of the subsets of sound sensors as either rib-aligned or intercostal space-aligned, based on an intensity of sound signals received at each of the subsets.

3. An acoustic sensing apparatus (12) as claimed in claim 2, wherein the controller (24) is operable to implement a localized sound pick-up using the arrangement (16) of sound sensors (18), and wherein the controller is adapted to localize sound pick-up based on the classifications of each of the subsets of sound sensors.

4. An acoustic sensing apparatus (12) as claimed in any of claims 1-3, wherein the apparatus is configured for sensing and processing heart-generated sound signals, lung generated sound signals, breathing sounds, and/or for sensing and processing subject vocal sounds.

5. An acoustic sensing apparatus (12) as claimed in claim 4 and in any of claims 2-3, wherein the controller (24) is adapted to classify a location, for example a subset (20) of sound sensors, as rib-aligned based on sensing a relative lower intensity sound signal at sensors (18) of that location or subset, and to classify a location, for example a subset, as intercostal space-aligned based on sensing a relative higher-intensity sound signal at sensors of that location or subset.

6. An acoustic sensing apparatus (12) as claimed in any of claims 1-5, wherein the apparatus further comprises at least one sound generator (36) adapted to generate sound signals for transmission into the chest, and wherein the one or more sound sensors (18) are adapted for sensing said generated sound signals, and optionally wherein
the controller (24) is adapted to classify a location, for example a subset (20) of sound sensors, as rib-aligned based on sensing a relative higher intensity sound signal at sensors (18) of that location or subset, and to classify a location, for example a subset, as intercostal space-aligned based on sensing a relative lower-intensity sound signal at sensors of that location or subset.

7. An acoustic sensing apparatus (12) as claimed in claim 6, wherein the sound generator (36) is adapted to generate sound signals comprising a spectrum or band of multiple sound frequencies.

8. An acoustic sensing apparatus (12) as claimed in any of claims 1 to 7, wherein the controller (24) is adapted to derive a sound signal intensity distribution in respect of a spatial area across which the arrangement of sound sensors is acoustically sensitive (16),
and wherein the controller (24) is further adapted to identify one or more sound intensity hotspots within the signal intensity distribution, the hotspots being areas within the intensity distribution exceeding a defined intensity threshold.

9. An acoustic sensing apparatus (12) as claimed in any of claims 2 to 8, wherein the sensing apparatus includes control means for controlling automated auscultation of the subject's chest using the arrangement (16) of sound sensors (18).

10. An acoustic sensing apparatus (12) as claimed in claim 9, wherein, responsive to the classification of the subsets of the arrangement, the controller (24) is adapted to activate the subsets (20) of the arrangement which are classified as intercostal space-aligned for performing auscultation, and/or to deactivate the subsets of the arrangement classified as rib-aligned.

11. An acoustic sensing apparatus (12) as claimed in claim 9 or 10, and as claimed in claim 8, wherein the controller (24) is adapted to extract from the sound signals sensed at the arrangement (16) of sound sensors (18) a localized sound signal corresponding to a location of the one or more identified hotspots within the intensity distribution.

12. An acoustic sensing apparatus (12) as claimed in any of claims 1 to 11, wherein at least the arrangement (16) of sound sensors (18) is integrated in a body-mountable patch (14), for mounting on a chest of the subject.

13. An acoustic sensing apparatus (12) as claimed in any of claims 1 to 12, further comprising an ultrasound sensing arrangement comprising one or more ultrasound transducers.

14. An acoustic sensing apparatus (12) as claimed in claim 13 and as claimed in claim 8, wherein the ultrasound sensing arrangement comprises an array of ultrasound transducers, and includes means for performing ultrasound beam steering using the array of ultrasound transducers, and wherein the transducer array is controlled to steer ultrasound signals toward one or more of the detected hotspots.

15. An acoustic sensing method (60) for sensing positions of ribs within a chest of a subject, comprising:
receiving (62) sound signals at a plurality of different locations across the chest of the subject using an arrangement (16) of one or more sound sensors (18); and
processing (64) the sound signals received at the plurality of different locations to classify each location as either rib-aligned or intercostal space-aligned, based on an intensity of sound signals received at the location.
